# EUROPEAN PATENT APPLICATION

(11) **EP 3 120 720 A1**
(43) Date of publication of application: **25.01.2017**
(21) Application number: 14886049.7
(22) Date of filing: 20.03.2014
(51) Int. Cl.: A24F 47/00

(54) **METHOD FOR PREVENTING A CHILD FROM ACCIDENTALLY PUFFING AN ELECTRONIC CIGARETTE**

(71) Applicant: Kimree Hi-Tech Inc, Road Town, Tortola (VG)
(72) Inventor: LIU, Qiuming, Shenzhen Guangdong 518000 (CN)
(74) Representative: Puschmann Borchert Bardehle Patentanwälte Partnerschaft mbB
(86) International application number: PCT/CN2014/073785
(87) International publication number: WO 2015/139272

(57) **Abstract**

A method for preventing a child from accidentally puffing an electronic cigarette, comprising: S0, awaiting unlock, and if an unlock signal is sent, then proceeding to step S1; S1, the electronic cigarette entering a state of normal operation; S2, timing; S3, determining whether a smoking signal is received within a first preset time; if so, proceeding to step S4; otherwise, the electronic cigarette entering a locked state and switching to step S0; S4, the electronic cigarette performing vaporization, and this time, after transmission of the smoking signal ends, switching to step S2. While the electronic cigarette is in the locked state, even if a child accidentally puffs the electronic cigarette, a non-smoking flow of air triggers the smoking switch, or a user accidentally activates the smoking switch, there will be no reaction of the electronic cigarette. If normal smoking is desired, one must first activate the unlock switch, causing the electronic cigarette to switch from the locked state to a state of normal operation; thus the probability of a child accidentally puffing or accidentally triggering the electronic cigarette is greatly reduced; furthermore, if the user does not smoke for a long period of time, the electronic cigarette automatically enters the locked state; the invention is automatic, convenient, and energy-saving.

## Description

### TECHNICAL FIELD

The present invention relates to electrical commodity, and more particularly relates to a method for preventing a child from accidentally puffing an electronic cigarette.

### BACKGROUND

An electronic cigarette comprises a battery pole and an atomizer. The battery pole comprises a battery used for providing the electronic cigarette working voltage. The atomizer comprises an electrical heating wire and an oil storage mechanism used for providing smoke oil for the electrical heating wire. The electronic cigarette in prior art senses the air flow which is produced by users' puffing via an air flow sensor. The detection of smoking state is realized by combining with the internal processing circuit, and the electronic cigarette will response when detecting smoking state. Specifically, the controlling module controls the battery to supply power for the electrical heating wire. The electrical heating wire heats to atomize the smoke oil in the oil storage mechanism to realize smoking.

The switch microphone or capacitance microphone is usually applied for the air flow sensor. The air flow sensor senses the air flow change to make the circuit work. However, when children play the electronic cigarette as a toy by mistake to puff, the electronic cigarette will produce smoke to influence the children's health. And it may cause safety problems. Besides, the electronic cigarette may be triggered when there is a non-smoking air flow or in the influence of the environmental factors such as temperature, resulting in wrong sense of the electronic cigarette.

There is another type of electronic cigarette, the air flow sensor is replaced by a touching switch, users touch the touching switch which is mounted at a particular area to realize sending a smoking signal to the internal processing circuit, then the internal processing circuit controls the battery to supply power for the electrical heating wire. Children may also play this type of electronic cigarette as a toy by mistake to puff. Besides, it is more easy to activate the touching switch by mistake in this type of electronic cigarette. For example, the touching switch is easy to touch when users take the electronic cigarette, and to touch the human body when the electronic cigarette is in the pocket.

Therefore, the defects in the prior art such as the electronic cigarette is easy to be puffed by children need to be improved.

### SUMMARY

The technical problem that the present invention will solve is to provide a method for preventing a child from accidentally puffing an electronic cigarette, aiming at the defect that electronic cigarettes are easy to be puffed by children in prior art.

The present invention provides a method for preventing a child from accidentally puffing an electronic cigarette, the electronic cigarette comprises an atomizing component used for atomizing smoke oil, a battery used for supplying power for the atomizing component, a smoking switch used for sending a smoking signal, and a controlling module, wherein the electronic cigarette further comprises an unlocking switch used for sending a unlocking signal to make the electronic cigarette enter a working state from a locking state, the battery, the atomizing component, the smoking switch and the unlocking switch are connected to the controlling module, respectively;
the method comprises:
S0, await unlocking;
when the electronic cigarette is in the locking state, the controlling module always controls the battery not to supply power for the atomizing component; if the unlocking switch is turned on to send the unlocking signal, then proceed to a step S1;
S1, the controlling module receives the unlocking signal and controls the electronic cigarette to enter the working state;
S2, time;
S3, determine whether the smoking signal is received within a first preset time; if so, proceed to a step
S4; otherwise, the electronic cigarette enters the locking state and switch to the step S0;
S4, the controlling module controls the battery to supply power for the atomizing component, and after transmission of the smoking signal, switch to the step S2.

In the method for preventing a child from accidentally puffing an electronic cigarette of the present invention, the electronic cigarette further comprises a warning module connected to the controlling module,

in the step S0, if turning on the smoking switch to send the smoking signal, proceed to a step S5; S5, the controlling module receives the smoking signal and controls the warning module to light or sound to warn users to turn on the unlocking switch.

In the method for preventing a child from accidentally puffing an electronic cigarette of the present invention, the warning module comprises a first LED light,
in the step S5, the first LED light warns users to turn on the unlocking switch by flashing;
the step S0 further comprises: the first LED light is always lighting or not lighting to instruct that the electronic cigarette is in the locking state;
the step S1 further comprises: the first LED light is always not lighting or lighting to instruct that the electronic cigarette is in the working state.

In the method for preventing a child from accidentally puffing an electronic cigarette of the present invention, the unlocking switch is further used for sending a first setting signal, in the step S2, if controlling module receives the first setting signal, proceed to a step S6:
S6, reset timing, reset the first preset time, and proceed to the step S2.

In the method for preventing a child from accidentally puffing an electronic cigarette of the present invention, the resetting the first preset time comprises:
S61, reset timing, controlling module waits for the unlocking switch to input time;
S62, unlocking switch inputs time, the controlling module saves the input time as the first preset time, proceed to the step S2.

In the method for preventing a child from accidentally puffing an electronic cigarette of the present invention, in the step S0, turning on the unlocking switch comprises: a touching path which is formed by users' touching actions is same as a preset touching path.

In the method for preventing a child from accidentally puffing an electronic cigarette of the present invention, the unlocking switch is further used for sending a second setting signal, in the step S2, if the controlling module receives the second setting signal, proceeding to a step S7:
S7, reset timing, reset the preset touching path, proceed to the step S2.

In the method for preventing a child from accidentally puffing an electronic cigarette of the present invention, the second setting signal is sent by the unlocking switch, the resetting the preset touching path comprises:
S71, reset timing, controlling module waits for the unlocking switch to input a touching path;
S72, the unlocking switch inputs a touching path, the controlling module saves the touching path as the preset touching path, proceed to the step S2.

In the method for preventing a child from accidentally puffing an electronic cigarette of the present invention, the unlocking switch comprises at least one touching key used for sensing touching and a capacitive touching sensing integrated chip used for sending the unlocking signal;
every touching key is connected to a corresponding touching input port of the capacitive touching sensing integrated chip, respectively, an output port of the capacitive touching sensing integrated chip is connected to the controlling module;
a quantity or a sequence of input touching actions in a second preset time forms the touching path.

In the method for preventing a child from accidentally puffing an electronic cigarette of the present invention, the controlling module comprises a microprocessor, a type of the microprocessor is SN8P2711B, the capacitive touching sensing integrated chip is a two-channel capacitive touching sensing integrated chip, a type of the two-channel capacitive touching sensing integrated chip is JG602, the touching key comprises a first touching key and a second touching key;
a TP0 pin and a TP1 pin of the two-channel capacitive touching sensing integrated chip are connected to the first touching key and the second touching key, respectively, a AHLB pin and a TOG pin of the two-channel capacitive touching sensing integrated chip are both vacant, a VDD pin and a VSS pin of the two-channel capacitive touching sensing integrated chip are connected to an anode and a cathode of the battery correspondingly, a TPQ0 pin and a TPQ1 pin of the capacitive touching sensing integrated chip are connected to a P4.4 pin and a P4.0 pin of the microprocessor correspondingly.

In the method for preventing a child from accidentally puffing an electronic cigarette of the present invention, a sequence of input touching actions in the second preset time forms the touching path; the preset touching path is:
firstly touch the first touching key, and touch the second touching key, outputting the high electrical level by the TPQ0 pin and the TPQ1 pin successively is the corresponding unlocking signal;
or firstly touch the second touching key, and then touch the first touching key, outputting the high electrical level by the TPQ1 pin and the TPQ0 pin successively is the corresponding un-locking signal.

In the method for preventing a child from accidentally puffing an electronic cigarette of the present invention, a quantity of input touching actions in the second preset time forms the touching path; the preset touching path is:
touch the first touching key and the second touching key in the second preset time, outputting the high electrical level by the TPQ1 pin and the TPQ0 pin in the second preset time is the corresponding unlocking signal.

In the method for preventing a child from accidentally puffing an electronic cigarette of the present invention, the electronic cigarette comprises a second LED light, in the step S4, the controlling module controlling the battery to supply power for the atomizing component further comprises: control the second LED light to light to instruct that the electronic cigarette is in smoking state.

In the method for preventing a child from accidentally puffing an electronic cigarette of the present invention, the atomizing component comprises an electrical heating wire, the controlling module comprises the microprocessor and a MOS tube, the smoking switch is an air flow sensor,
a source of the MOS tube is connected to the cathode of the battery, a drain of the MOS tube is connected to the anode of the battery via the electrical heating wire, a grid of the MOS tube is connected to the microprocessor; a signal output end of the air flow sensor is connected to the microprocessor,
the smoking signal is a voltage signal which is output from the signal output end when the air flow sensor senses an air flow, in the step S5, the controlling module controlling the battery (1) to supply power for the atomizing component comprises: the microprocessor outputting high level to the grid of the MOS tube, the MOS tube conducts, the battery and the electrical heating wire are electrically connected.

The beneficial effects of implementing the method for preventing a child from accidentally puffing an electronic cigarette are: a locking state is added. When the electronic cigarette is in the locking state, the controlling module always controls the battery not to supply power for the atomizing component. So even if children puff the electronic cigarette by mistake, the nonsmoking air flow triggers the smoking switch or the users accidentally turn on the smoking switch, the electronic cigarette will not response. If users want to smoke, they must turn on the unlocking switch first to switch the electronic cigarette in the locking state to working state. Therefore, the possibility of accidentally puffing or triggering the electronic cigarette by children can be greatly reduced by using the method of the present invention. Meantime, the electronic cigarette can automatically enter the locking state when users do not puff the electronic cigarette for a long time. It is convenient and can save energy.

### BRIEF DESCRIPTION OF THE DRAWINGS

The invention will be further described with reference to the accompanying drawings and embodiments in the following.
- Fig. 1: is a flow diagram of the first embodiment of the method for preventing a child from accidentally puffing an electronic cigarette of the present invention;
- Fig. 2: is a structural diagram of the first embodiment of the method for preventing a child from accidentally puffing an electronic cigarette of the present invention;
- Fig. 3: is a circuit diagram of the first embodiment of the electronic cigarette in Figure 2;
- Fig. 4: is a circuit diagram of the second embodiment of the electronic cigarette in Figure 2;
- Fig. 5: is a flow diagram of the second embodiment of the method for preventing a child from accidentally puffing an electronic cigarette of the present invention;
- Fig. 6: is a structural diagram of the second embodiment of the method for preventing a child from accidentally puffing an electronic cigarette of the present invention;
- Fig. 7: is a circuit diagram of the better embodiment of the electronic cigarette in Figure 6;
- Fig. 8: is a flow diagram of the third embodiment of the method for preventing a child from accidentally puffing an electronic cigarette of the present invention.

### DETAILED DESCRIPTION OF THE PREFERRED EMBODIMENT

Implementing the method for preventing a child from accidentally puffing an electronic cigarette, a locking state is added. When the electronic cigarette is in the locking state, the controlling module always controls the battery not to supply power for the atomizing component. So even if children puff the electronic cigarette by mistake, the nonsmoking air flow triggers the smoking switch or the users accidentally turn on the smoking switch, the electronic cigarette will not response. If users want to smoke, they must turn on the unlocking switch first to switch the electronic cigarette in the locking state to working state. Therefore, the possibility of accidentally puffing or triggering the electronic cigarette by children can be greatly reduced by using the method of the present invention. Meantime, the electronic cigarette can automatically enter the locking state when users do not puff the electronic cigarette for a long time. It is convenient and can save energy.

In order to better understand the technical features, purpose and effect of the present invention, the preferred embodiment will be described in detail in the following.

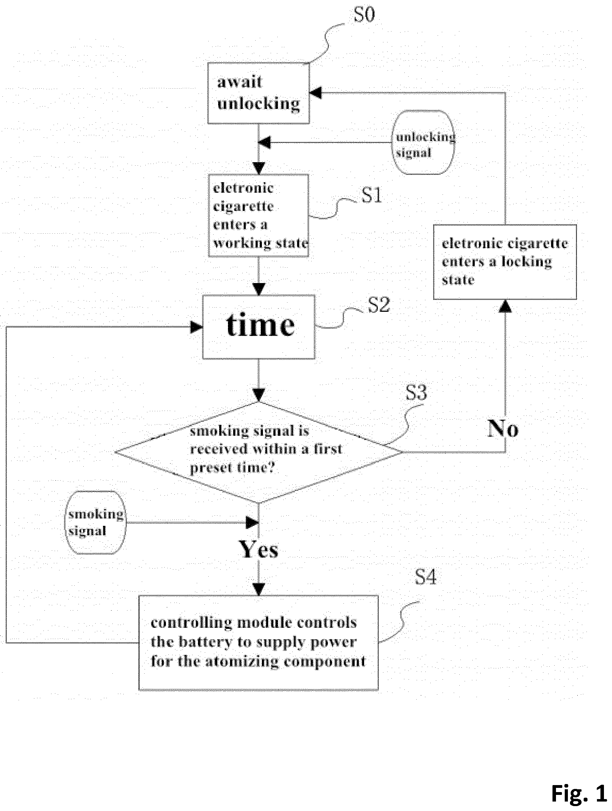

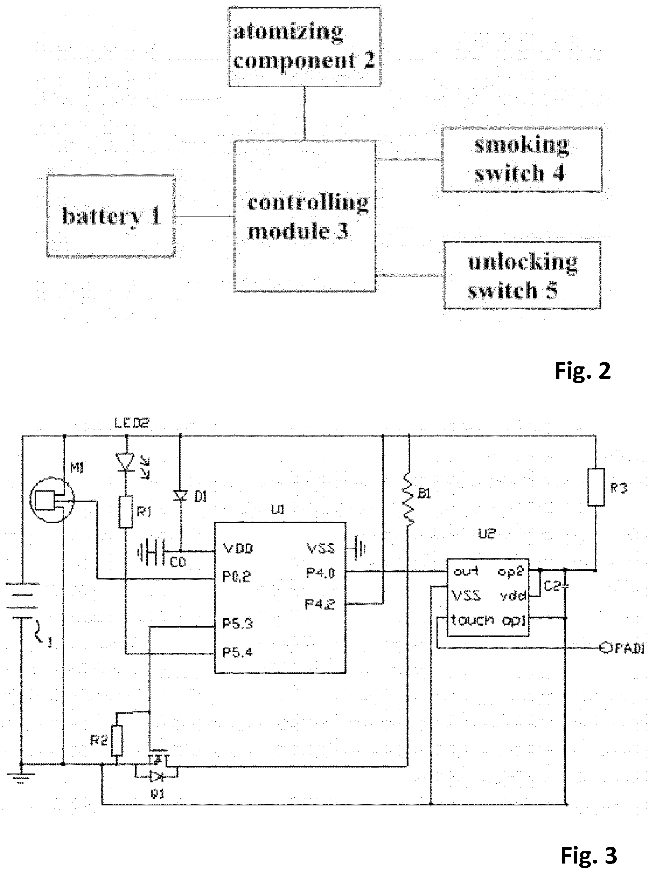

Figure 1 is a flow diagram of the first embodiment of the method for preventing a child from accidentally puffing an electronic cigarette of the present invention; figure 2 is a structural diagram of the first embodiment of the method for preventing a child from accidentally puffing an electronic cigarette of the present invention.

Referred to figure 2 first, the electronic cigarette comprises an atomizing component 2 used for atomizing smoke oil, a battery 1 used for supplying power for the atomizing component 2, a smoking switch 4 used for sending a smoking signal, and a controlling module 3, wherein the electronic cigarette further comprises an unlocking switch 5 used for sending a unlocking signal to make the electronic cigarette enter a working state from a locking state, the battery 1, the atomizing component 2, the smoking switch 4 and the unlocking switch 5 are connected to the controlling module 3, respectively.

In the first embodiment, the method comprises:
S0, await unlocking,
when the electronic cigarette is in the locking state, the controlling module 3 always controls the battery 1 not to supply power for the atomizing component 2.

In this state, if the smoking switch 4 sends the smoking signal, the controlling module 3 will not control the battery 1 to supply power for the atomizing component 2. If users need to smoke, they must turn on the unlocking switch 5 first. Once the unlocking switch 5 is turned on, proceed to step S1.

S1, the controlling module 3 receives the unlocking signal and controls the electronic cigarette to enter a working state.

Once the electronic cigarette enters the working state, the controlling module 3 will automatically block the unlocking signal sent by the unlocking switch 5. The unlocking signal is only effective when the electronic cigarette is in the locking state. It is worth noting that the unlocking signal is blocked, but the other signals (the first setting signal and the second setting signal) sent by the unlocking switch 5 in the third embodiment will not be blocked.
S2, time.
S3, determine whether the smoking signal is received within a first preset time; if so, proceed to step S4; otherwise, the electronic cigarette enters the locking state and switch to step S0.
S4, the controlling module 3 controls the battery 1 to supply power for the atomizing component 2, and after transmission of the smoking signal, switch to step S2.

In the step S0, the unlocking switch 5 can a normal key switch. Advantageously, the unlocking switch 5 comprises at least one touching key used for sensing touching and a capacitive touching sensing integrated chip used for sending the unlocking signal; every touching key is connected to a corresponding touching input port of the capacitive touching sensing integrated chip, respectively, an output port of the capacitive touching sensing integrated chip is connected to the controlling module 3.

Correspondingly, in the step S0, turning on the unlocking switch 5 comprises: a touching path which is formed by users' touching actions is same as a preset touching path. In this embodiment, the touching path is defined as: a quantity or a sequence of input touching actions in a second preset time forms the touching path. The preset touching path, the first preset time and the second preset time are written in the software of the controlling module 3 by the manufacturers. Users can rewrite, the details will be described in the third embodiment.

If time is not restricted in the second preset time, the input touching actions will be accumulated. For example, if there are three touching keys 1, 2, 3, the preset touching path is: touching 1, 2, 3 successively. If users input 1,2 in turn for the first time, and then do not input, if time is not restricted in the second preset time, the controlling module 3 will always wait for users to input subsequent touching actions. If users input 1, 2, 3 for the second time after a period of time, the controlling module 3 will add the second touching actions to the first touching actions. That is to say, the users' second input is correct, the unlocking switch 5 should be turned on. But the controlling module 3 judges that the users input 1, 2, 1, 2, 3. So the users' input is wrong when inputting 1 for the second time.

There are two conditions that the electronic cigarette in the step S2 switches to automatically locking: One is after users turn on the unlocking switch 5, if the smoking switch 4 is not turned on in the first preset time, the electronic cigarette will enter the locking state from the working state; if the smoking switch 4 is turned on in the first preset time, then proceed to the step S4, the smoking signal will be responded.

The other is after users turn on the unlocking switch 5, the smoking switch 4 is turned on for at least one time. The timing is proceeded from the step S4. If users do not turn on the smoking switch 4 in the preset time after the smoking switch 4 sends the smoking signal, the electronic cigarette will switch to the locking state from the working state. And if the time between turning on the smoking switch 4 and finishing sending the smoking signal is in the preset time, then proceed to the step S4 again, the smoking signal will be responded.

The process of the method will be described combined with the specific circuit structure of the electronic cigarette in the following.

Figure 3 is a circuit diagram of the first embodiment of the electronic cigarette in Figure 2.

In the first embodiment, the capacitive touching sensing integrated chip is a single-channel capacitive touching sensing integrated chip U2, the type of the single-channel capacitive touching sensing integrated chip U2 is AR101. So only one touching key can be effectively detected. The corresponding touching path is: touching the touching key.

The atomizing component 2 comprises an electrical heating wire B1. The controlling module 3 comprises a microprocessor U1 and an N-type MOS tube Q1, the type of the microprocessor U1 is SN8P2711B. The smoking switch 4 is an air flow sensor M1. The electronic cigarette further comprises a second LED light LED2.

A source of the MOS tube Q1 is connected to the cathode of the battery 1, a drain of the MOS tube Q1 is connected to the anode of the battery 1 via the electrical heating wire B1, and a grid of the MOS tube Q1 is connected to the P5.3 pin of the microprocessor U1. The grid of the MOS tube Q1 is connected to the source of the MOS tube Q1 via the resistance R2. A signal output end of the air flow sensor M1 is connected to the P0.2 pin of the microprocessor U1. The power supply end and the grounding end of the air flow sensor M1 are connected to the anode and the cathode of the battery 1 correspondingly. The VDD pin of the microprocessor U1 is grounded via the capacitance C0. The VDD pin is further connected to the cathode of the diode D1. The anode of the diode D1 is connected to the anode of the battery 1. The anode of the second LED light LED2 is connected to the anode of the battery 1. The cathode of the second LED light LED2 is connected to the P5.4 pin of the microprocessor U1 via the resistance R1. The VSS pin of the microprocessor U1 is grounded.

The out pin of the single-channel capacitive touching sensing integrated chip U2 is connected to the P4.0 pin of the microprocessor U1, the out pin is used to output the unlocking signal, the touch pin is connected to the first touching key PAD1. The vdd pin and the op2 pin are in parallel and connected to the anode of the battery 1 via the capacitance R3. Thus, the out output is a hold mode. The vdd pin is grounded via the capacitance C2. The op1 pin and the VSS pin are grounded. So the out output is a high level.

In the step S0:
When the electronic cigarette is in the locking state, the P5.3 pin of the microprocessor U1 always output low level, so the MOS tube Q1 keeps cut-off. At this time, no matter whether the P0.2 pin of the microprocessor U1 receives the voltage signal from the air flow sensor M1, the P5.3 pin will not output high level. The P5.4 pin of the microprocessor U1 keeps outputting high level. The second LED light LED2 do not light.

If users touch the first touching key PAD1, the unlocking signal that is output by the out pin of U2 is: change the output level.

In the step S1: the P4.0 of the microprocessor U1 receives the changed level to control the electronic cigarette to enter the working state.

In the step S2: the timing can be realized by a timer separated from the microprocessor U1. The preferred one in this embodiment is to use the internal timer TC0 or TC1 of the microprocessor U1.

If users inhale, the signal output end of the air flow sensor M1 outputs the voltage signal as the smoking signal and sends to the P0.2 pin of the air flow sensor M1.

In the step S4: the P5.3 pin of the air flow sensor M1, the MOS tube conducts, the battery and the electrical heating wire B1 are electrically connected. The electrical heating wire B1 heats to atomizer the smoke oil. Meantime, the P5.4 pin of the electrical heating wire B1 outputs low lever, the second LED light LED2 lights to instruct that the electronic cigarette is in the smoking state.

Controlling the MOS tube Q1 to conduct is to conduct for a period of time then recover the cut-off of the MOS tube Q1. The moment of retime in the step S2 can be the moment that the P0.2 pin completes to receive the smoking signal or the moment that the MOS tube Q1 completes to conduct.

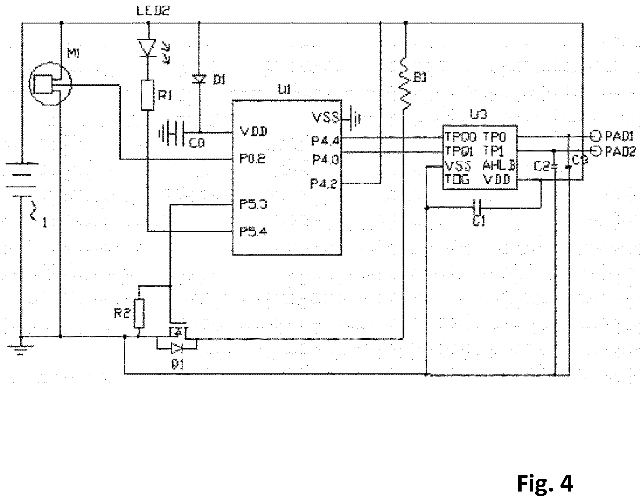

Figure 4 is a circuit diagram of the second embodiment of the electronic cigarette in Figure 2.

The difference of the second embodiment with the first embodiment is the unlocking switch 5. The capacitive touching sensing integrated chip in the second embodiment is a two-channel capacitive touching sensing integrated chip U3. The type of the two-channel capacitive touching sensing integrated chip U3 is JG602. The touching key comprises a first touching key PAD1 and a second touching key PAD2.

A TP0 pin and a TP1 pin of the two-channel capacitive touching sensing integrated chip U3 are connected to the first touching key PAD1 and the second touching key PAD2, respectively. The TP0 pin and TP1 pin are grounded via the filter capacitor C3 and the filter capacitor C2. A AHLB pin and a TOG pin of the two-channel capacitive touching sensing integrated chip U3 are both vacant, thus the effective output is the high level and the output level is not locked. A VDD pin and a VSS pin of the two-channel capacitive touching sensing integrated chip U3 are connected to an anode and a cathode of the battery 1 correspondingly, the VDD pin is grounded via the filter capacitor C1. A TPQ0 pin and a TPQ1 pin of the capacitive touching sensing integrated chip U3 are connected to a P4.4 pin and a P4.0 pin of the microprocessor U1 correspondingly.

If the first touching key PAD1 is touched, the TP0 pin outputs high level. If the second touching key PAD2 is touched, the TP1 pin outputs high level. The level signals that is output by the TP0 pin and the TP1 pin are sent to the P4.4 pin and the P4.0 pin of the microprocessor U1. Thus, the microprocessor U1 judges whether the touching path is the preset touching path according to the received level signal.

According to the definition of the touching path: a quantity or a sequence of input touching actions in a second preset time forms the touching path. The specific definition of the touching path and the set of the preset touching path are provided in the following:

For example, the sequence of input touching actions in a second preset time forms the touching path. In this condition, the set of the preset touching path is:
Firstly touch the first touching key PAD1, and then touch the second touching key PAD2, the corresponding locking signal in high electrical level is outputted from the TPQ0 pin and the TPQ1 successively.

Or firstly touch the second touching key PAD2, and then touch the first touching key PAD1, the corresponding locking signal in high electrical level is outputted from the TPQ1 pin and the TPQ0 successively.

And for example, a quantity of input touching actions in a second preset time forms the touching path, the preset touching path is: touch the first touching key PAD1 and the second touching key PAD2 in the second preset time, the locking signal in high electrical level is outputted from the TPQ0 pin and the TPQ1 in the second preset time. That is to say, at this time two touching keys should be touched in the second preset time, which means unlocking. It is not related to the sequence but the quantity of the touched touching keys.

To be clear, the touching path besides the above ones can be the permutation and combination of the quantity and the sequence of the touching keys. For example, touch the touching key PAD1 twice, then touch the touching key PAD2 once, then touch the touching key PAD1 once, as long as the touching path is formed by the quantity and the sequence of the touching keys. The quantity of the touching key and the choice of the capacitive touching sensing integrated chip are not restricted in above ones, such as the four-channel capacitive touching sensing integrated chip and the six-channel capacitive touching sensing integrated chip. The capacitive touching sensing integrated chip can be replaced by the resistive touching sensing integrated chip and normal key switch.

To be further clear, the arrangement of the touching key is not restricted. For example, the touching keys can be arranged on the outer wall of the electronic cigarette in parallel with the axis of electronic cigarette or on the corresponding location of the side wall of the electronic cigarette.

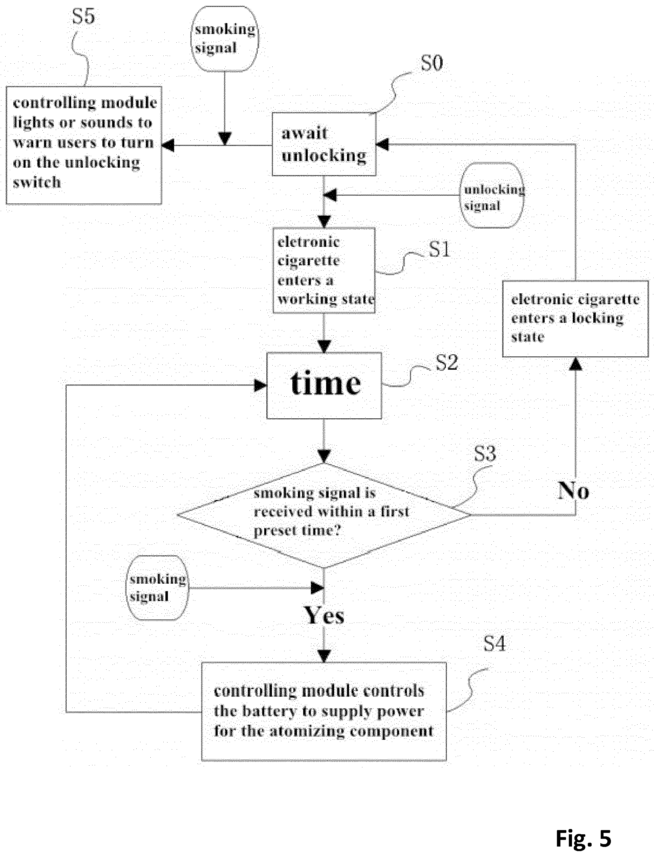

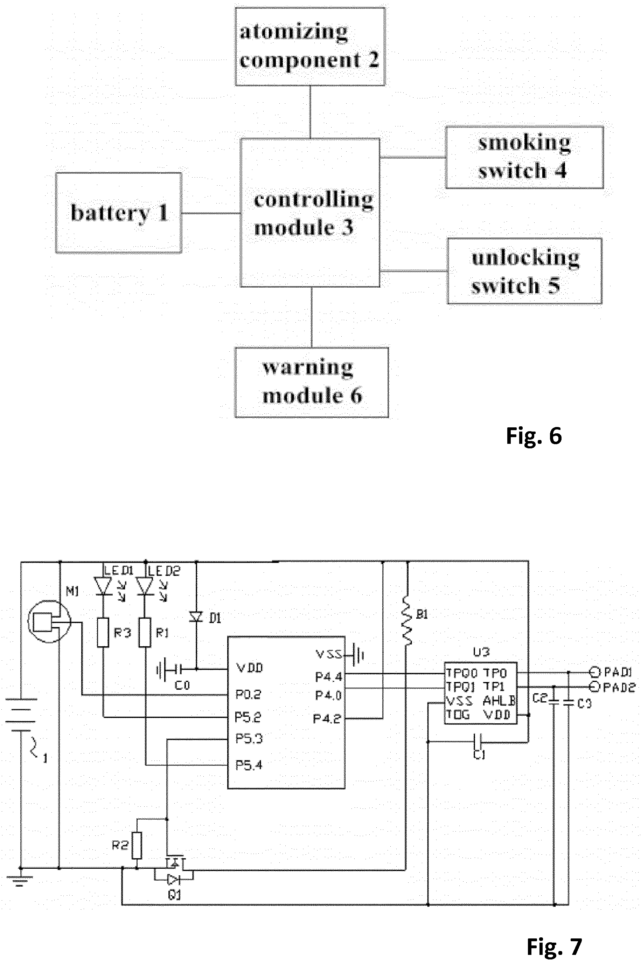

Figure 5 is a flow diagram of the second embodiment of the method for preventing a child from accidentally puffing an electronic cigarette of the present invention. Figure 6 is a structural diagram of the second embodiment of the method for preventing a child from accidentally puffing an electronic cigarette of the present invention.

In comparison with the first embodiment, the electronic cigarette of the second embodiment is added a warning module 6. The warning module 6 is connected to the controlling module 3.

In the step S0, if the smoking switch 4 is turned on, then proceed to the step S5;
S5, the controlling module 3 receives the smoking signal and controls the warning module 6 to light or sound to warn users to turn on the unlocking switch 5.

Specifically, Figure 7 is a circuit diagram of the better embodiment of the electronic cigarette in Figure 6.

In comparison with the first embodiment and the second embodiment, a warning module 6 is added, the warning module 6 comprises a first LED light LED1.

In the step S5, the first LED light LED1 warns the users to turn on the unlocking switch 5 by flashing.

And, the step S0 further comprises: the first LED light LED1 is always lighting or not lighting to instruct that the electronic cigarette is in the locking state; the step S1 further comprises: the first LED light LED1 is always not lighting or lighting to instruct that the electronic cigarette is in the working state.

According to living habits, lighting is generally used to instruct effectiveness. Advantageously, the first LED light LED1 is not lighting to instruct that the electronic cigarette is in the locking state; the step S1 further comprises: the first LED light LED1 is always lighting to instruct that the electronic cigarette is in the working state.

In order to improve the users' feeling of the electronic cigarette, the above first preset time and preset touching path can be reset by the users. For example, some uses think that the default first preset time is too short, they want to extend the "standby" time. Or users think the default preset touching path is rigid, they want to set a personal way to turn on the unlocking switch 5. Or users want to set a way to turn on the unlocking switch 5 that the unlocking switch 5 can only be unlocked by themselves to avoid that avoid that other people use their own electronic cigarette.

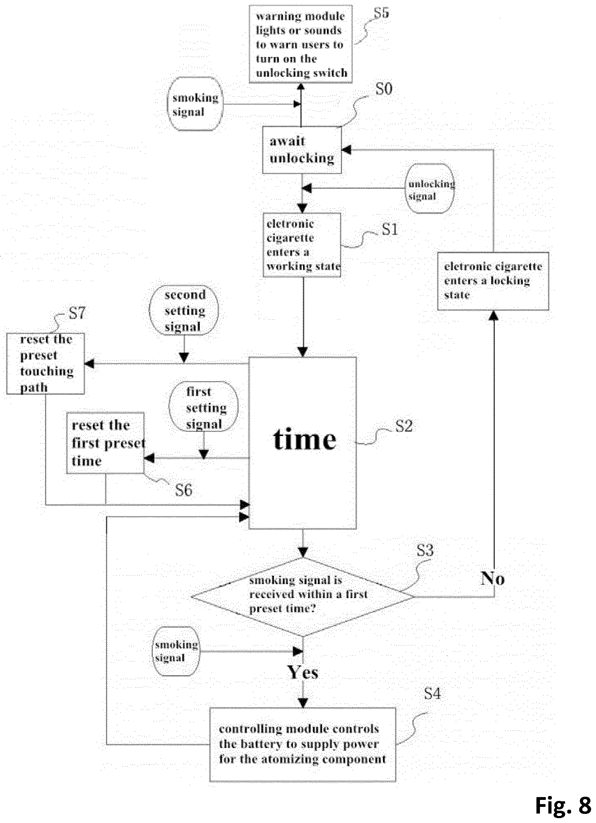

Figure 8 is referred to introduce this embodiment in the following.

The difference of this embodiment with the second embodiment is: in the process of timing in the step S2: if the controlling module receives the first setting signal, then proceed to the step S6; if the controlling module receives the second setting signal, then proceed to the step S7:
S6, reset timing, reset the first preset time again, and proceed to the step S2.
S7, reset timing, reset the preset touching path again, and proceeds to the step S2.

The activation of the function of resetting must be executed when the electronic cigarette is in the unlocking state. That is to say, in the process of timing, changing the set by other people can be avoided. This function can be realized by specialized setting switch. In order to save components in the present invention, this function is realized by the unlocking switch 5. Using the structure of the electronic cigarette in the second embodiment, when the first touching key PAD1 is long pressed in the embodiment, the long-time level signal that is output by the TP0 pin is the first setting signal. When the second touching key PAD2 is long pressed, the long-time level signal that is output by the TP1 pin is the second setting signal.

To be clear, the definition of the first setting signal and the second setting signal is not restricted in the present invention. For example, when the two touching keys are long pressed at the same time, the long-time level signal that the TP0 and the TP1 output is the first setting signal and the second setting signal.

The step S6 comprises:
S61, reset timing, controlling module 3 waits for the unlocking switch 5 to input time; the time of waiting for the inputting is determined when leaving the factory according to the needs, such as 5s. If users do not input then cancel the set, and proceed to the step 2;
S62, unlocking switch 5 inputs time, the controlling module 3 saves the input time as the first preset time, reset timing, proceed to the step S2.

The time input can be realized by counting the times of the touching of the touching key. Every time when setting, the first preset time is based on the last saved one to increase or reduce. For example, touching the first touching key PAD1 represents increasing 3s, touching the second touching key PAD2 represents reducing 3s. After setting, by outputting a signal which is same as the first setting signal again, this signal can be as the instructing signal that instructs finishing setting and saving.

The step S7 comprises:
S71, the controlling module 3 waits for the unlocking switch 5 to input the touching path; the detail is same as the S61.
S72, the unlocking switch 5 inputs the touching path, the controlling module 3 saves the input touching path as the preset touching path, reset timing, proceed to the step S2.

The step S72 is similar as the step S62. It is worth noting that if users want to set the quantity of the touching actions as the touching path, namely the preset touching path is the quantity of the touched touching keys, they must simultaneously touch the touching keys that need to be set at some time. For example, for the electronic cigarette with 6 touching keys, the default preset touching path is touching the 6 touching keys simultaneously. But users think it is troublesome, and they want to set simultaneously touching two touching keys to turn on the unlocking switch to send out the unlocking signal. After actuating this function, users need to keep touching the two touching keys simultaneously when inputting the touching path. If users touch the two touching keys one after another, it will be considered that the preset touching path is set according to the sequence. For the electronic cigarette with one touching key, the method based on the quantity and the method based on the sequence are the same.

Advantageously, after users finish setting and saving, users need to input again to ensure they memorize the input.

In the same way, the second preset time can be reset. The set is similar as the first preset time, the details will not be discussed.

In conclusion, for the method for preventing a child from accidentally puffing an electronic cigarette of the present invention, a locking state is added. When the electronic cigarette is in the locking state, the controlling module always controls the battery not to supply power for the atomizing component. So even if children puff the electronic cigarette by mistake, the nonsmoking air flow triggers the smoking switch or the users accidentally turn on the smoking switch, the electronic cigarette will not response. If users want to smoke, they must turn on the unlocking switch first to switch the electronic cigarette in the locking state to working state. Therefore, the possibility of accidentally puffing or triggering the electronic cigarette by children can be greatly reduced by using the method of the present invention. Meantime, the electronic cigarette can automatically enter the locking state when users do not puff the electronic cigarette for a long time. It is convenient and can save energy.

Combining with the accompanying drawings, embodiments of the present invention are described. However, the present invention is not limited by the above embodiments, which means that the above specific embodiments are only schematic, rather than restrictive. It should be understood that, in the inspiration of the present invention, those skilled in the art who appreciate and realize all or part of the process in above embodiments may make many modifications or alternatives, without going beyond the purpose and the scope the claims intend to protect of the present application. All these belong to the protection of the present invention.

## Claims

1. A method for preventing a child from accidentally puffing an electronic cigarette, the electronic cigarette comprises an atomizing component (2) used for atomizing smoke oil, a battery (1) used for supplying power for the atomizing component (2), a smoking switch (4) used for sending a smoking signal, and a controlling module (3), wherein the electronic cigarette further comprises an unlocking switch (5) used for sending a unlocking signal to make the electronic cigarette enter a working state from a locking state, the battery (1), the atomizing component (2), the smoking switch (4) and the unlocking switch (5) are connected to the controlling module (3), respectively;
the method comprises:
S0, await unlocking;
when the electronic cigarette is in the locking state, the controlling module (3) always controls the battery (1) not to supply power for the atomizing component (2); if the unlocking switch (5) is turned on to send the unlocking signal, then proceed to a step S1;
S1, the controlling module (3) receives the unlocking signal and controls the electronic cigarette to enter the working state;
S2, time;
S3, determine whether the smoking signal is received within a first preset time; if so, proceed to a step S4; otherwise, the electronic cigarette enters the locking state and switch to the step S0;
S4, the controlling module (3) controls the battery (1) to supply power for the atomizing component (2), and after transmission of the smoking signal, switch to the step S2.

2. The method for preventing a child from accidentally puffing an electronic cigarette according to claim 1, wherein the electronic cigarette further comprises a warning module (6) connected to the controlling module (3), in the step S0, if turning on the smoking switch (4) to send the smoking signal, proceed to a step S5;
S5, the controlling module (3) receives the smoking signal and controls the warning module (6) to light or sound to warn users to turn on the unlocking switch (5).

3. The method for preventing a child from accidentally puffing an electronic cigarette according to claim 2, wherein the warning module (6) comprises a first LED light (LED1),
in the step S5, the first LED light (LED1) warns users to turn on the unlocking switch (5) by flashing;
the step S0 further comprises: the first LED light (LED1) is always lighting or not lighting to instruct that the electronic cigarette is in the locking state;
the step S1 further comprises: the first LED light (LED1) is always not lighting or lighting to instruct that the electronic cigarette is in the working state.

4. The method for preventing a child from accidentally puffing an electronic cigarette according to claim 1, wherein the unlocking switch (5) is further used for sending a first setting signal, in the step S2, if controlling module (3) receives the first setting signal, proceed to a step S6:
S6, reset timing, reset the first preset time, and proceed to the step S2.

5. The method for preventing a child from accidentally puffing an electronic cigarette according to claim 4, wherein the resetting the first preset time comprises:
S61, reset timing, controlling module (3) waits for the unlocking switch (5) to input time;
S62, unlocking switch (5) inputs time, the controlling module (3) saves the input time as the first preset time, proceed to the step S2.

6. The method for preventing a child from accidentally puffing an electronic cigarette according to claim 1, wherein in the step S0, turning on the unlocking switch (5) comprises: a touching path which is formed by users' touching actions is same as a preset touching path.

7. The method for preventing a child from accidentally puffing an electronic cigarette according to claim 6, wherein the unlocking switch (5) is further used for sending a second setting signal, in the step S2, if the controlling module (3) receives the second setting signal, proceed to a step S7:
S7, reset timing, reset the preset touching path, proceed to the step S2.

8. The method for preventing a child from accidentally puffing an electronic cigarette according to claim 7, wherein the second setting signal is sent by the unlocking switch (5), the resetting the preset touching path comprises:
S71, reset timing, controlling module (3) waits for the unlocking switch (5) to input a touching path;
S72, the unlocking switch (5) inputs a touching path, the controlling module (3) saves the touching path as the preset touching path, proceed to the step S2.

9. The method for preventing a child from accidentally puffing an electronic cigarette according to claim 6, wherein the unlocking switch (5) comprises at least one touching key used for sensing touching and a capacitive touching sensing integrated chip used for sending the unlocking signal;
every touching key is connected to a corresponding touching input port of the capacitive touching sensing integrated chip, respectively, an output port of the capacitive touching sensing integrated chip is connected to the controlling module (3);
a quantity or a sequence of input touching actions in a second preset time forms the touching path.

10. The method for preventing a child from accidentally puffing an electronic cigarette according to claim 9, wherein the controlling module (3) comprises a microprocessor (U1), a type of the microprocessor (U1) is SN8P2711 B, the capacitive touching sensing integrated chip is a two-channel capacitive touching sensing integrated chip (U3), a type of the two-channel capacitive touching sensing integrated chip (U3) is JG602, the touching key comprises a first touching key (PAD1) and a second touching key (PAD2);
a TP0 pin and a TP1 pin of the two-channel capacitive touching sensing integrated chip (U3) are connected to the first touching key (PAD1) and the second touching key (PAD2), respectively, a AHLB pin and a TOG pin of the two-channel capacitive touching sensing integrated chip (U3) are both vacant, a VDD pin and a VSS pin of the two-channel capacitive touching sensing integrated chip (U3) are connected to an anode and a cathode of the battery (1) correspondingly, a TPQ0 pin and a TPQ1 pin of the capacitive touching sensing integrated chip (U3) are connected to a P4.4 pin and a P4.0 pin of the microprocessor (U1) correspondingly.

11. The method for preventing a child from accidentally puffing an electronic cigarette according to claim 10, wherein a sequence of input touching actions in the second preset time forms the touching path; the preset touching path is:
firstly touch the first touching key (PAD1), and then touch the second touching key (PAD2), outputting the high electrical level by the TPQ0 pin and the TPQ1 pin successively is the corresponding unlocking signal;
or firstly touch the second touching key (PAD2), and touch the first touching key (PAD1), outputting the high electrical level by the TPQ1 pin and the TPQ0 pin successively is the corresponding unlocking signal.

12. The method for preventing a child from accidentally puffing an electronic cigarette according to claim 10, wherein a quantity of input touching actions in the second preset time forms the touching path; the preset touching path is:
touch the first touching key (PAD1) and the second touching key (PAD2) in the second preset time, outputting the high electrical level by the TPQ1 pin and the TPQ0 pin in the second preset time is the corresponding unlocking signal.

13. The method for preventing a child from accidentally puffing an electronic cigarette according to claim 1, wherein the electronic cigarette comprises a second LED light (LED2), in the step S4, the controlling module (3) controlling the battery (1) to supply power for the atomizing component (2) further comprises: control the second LED light (LED2) to light to instruct that the electronic cigarette is in smoking state.

14. The method for preventing a child from accidentally puffing an electronic cigarette according to claim 1, wherein the atomizing component (2) comprises an electrical heating wire (B1), the controlling module (3) comprises the microprocessor (U1) and a MOS tube (Q1), the smoking switch (4) is an air flow sensor (M1),
a source of the MOS tube (Q1) is connected to the cathode of the battery (1), a drain of the MOS tube (Q1) is connected to the anode of the battery (1) via the electrical heating wire (B1), a grid of the MOS tube (Q1) is connected to the microprocessor (U1); a signal output end of the air flow sensor (M1) is connected to the microprocessor (U1), the smoking signal is a voltage signal which is output from the signal output end when the air flow sensor (M1) senses an air flow, in the step S5, the controlling module (3) controlling the battery (1) to supply power for the atomizing component (2) comprises: the microprocessor (U1) outputting high level to the grid of the MOS tube (Q1), the MOS tube (Q1) conducts, the battery (1) and the electrical heating wire (B1) are electrically connected.
